# EUROPEAN PATENT SPECIFICATION

(11) **EP 4 084 740 B1**
(45) Date of publication and mention of the grant of the patent: **11.02.2026**
(21) Application number: 21712362.9
(22) Date of filing: 23.02.2021
(51) Int. Cl.: A61F 2/24

(54) **HYBRID FRAME FOR PROSTHETIC HEART VALVE**
HYBRIDRAHMEN FÜR HERZKLAPPENPROTHESE
BÂTI HYBRIDE POUR VALVE CARDIAQUE PROTHÉTIQUE

(30) Priority: 24.02.2020 US 202062980981 P
(43) Date of publication of application: 09.11.2022
(73) Proprietor: Edwards Lifesciences Corporation, Irvine, CA 92614-5688 (US)
(72) Inventor: NIR, Noam, 30889 Caesarea (IL); GOLDBERG, Eran, 30889 Caesarea (IL); BUKIN, Michael, 30889 Caesarea (IL)
(74) Representative: Venner, Julia Ann
(86) International application number: PCT/US2021/019254
(87) International publication number: WO 2021/173561

(56) References cited:
- EP-A1- 2 641 569
- EP-A1- 3 295 898
- WO-A2-2010/104638
- US-A1- 2019 060 057

## Description

### CROSS REFERENCE TO RELATED APPLICATION

This application claims the benefit of United States Provisional Application Serial No. 62/980,981, entitled HYBRID FRAME FOR PROSTHETIC HEART VALVE, filed on February 24, 2020.

### FIELD

The present disclosure relates to implantable, mechanically expandable prosthetic devices, such as prosthetic heart valves, and to methods and delivery assemblies for, and including, such prosthetic devices.

### BACKGROUND

The human heart can suffer from various valvular diseases. These valvular diseases can result in significant malfunctioning of the heart and ultimately require repair of the native valve or replacement of the native valve with an artificial valve. There are a number of known repair devices (e.g., stents) and artificial valves, as well as a number of known methods of implanting these devices and valves in humans. Percutaneous and minimally-invasive surgical approaches are used in various procedures to deliver prosthetic medical devices to locations inside the body that are not readily accessible by surgery or where access without surgery is desirable. In one specific example, a prosthetic heart valve can be mounted in a crimped state on the distal end of a delivery apparatus and advanced through the patient's vasculature (e.g., through a femoral artery and the aorta) until the prosthetic heart valve reaches the implantation site in the heart. The prosthetic heart valve is then expanded to its functional size, for example, by inflating a balloon on which the prosthetic valve is mounted, actuating a mechanical actuator that applies an expansion force to the prosthetic heart valve, or by deploying the prosthetic heart valve from a sheath of the delivery apparatus so that the prosthetic heart valve can self-expand to its functional size.

Prosthetic heart valves that rely on a mechanical actuator for expansion can be referred to as "mechanically expandable" prosthetic heart valves. Prosthetic heart valves that can expand without the use of a mechanical actuator or balloon can be referred to as "self-expanding" prosthetic heart valves. Each type of prosthetic heart valve can include various advantages.

Despite the recent advancements in percutaneous valve technology, there remains a need for improved transcatheter heart valves and delivery devices for such valves. EP 2 641 569 Al discloses a collapsible valve prosthesis e.g. mitral valve prosthesis that has over-annular portion that is extended collar-like over annulus of natural valve site at inflow side of blood flow lumen defined by tubular intra-annular portion

### SUMMARY

In a representative embodiment, an implantable prosthetic device can comprise a hybrid frame movable between a radially compressed configuration and a radially expanded configuration. The hybrid frame can comprise a mechanically-expandable first sub-frame comprising a plurality of struts pivotably coupled to one another and a self-expanding second sub-frame coupled to the first sub-frame. When the hybrid frame is in the expanded configuration, the second sub-frame can be configured to resist radial compression of the frame.

In another representative embodiment, an implantable prosthetic device can comprise a radially compressible and expandable frame. The frame can comprise a mechanically expandable first sub-frame comprising one or more expansion mechanisms configured to move the first sub-frame between a radially compressed configuration and a radially expanded configuration, and a self-expanding second sub-frame coupled to the first sub-frame. The second sub-frame can be configured to exert a radially outwardly directed force on the first sub-frame to prevent radial compression of the frame from the expanded configuration.

In still another representative embodiment, an implantable prosthetic device comprises a hybrid frame movable between a radially compressed configuration and a radially expanded configuration. The hybrid frame comprises a mechanically-expandable first sub-frame comprising a first set of struts pivotably coupled to one another, each strut of the first set of struts comprising a plurality of apertures extending through a thickness of the strut, and a self-expanding second sub-frame comprising a second set of struts, each strut of the second set of struts comprising a protrusion extending from a radially outer surface of the strut. The first and second sub-frames are coupled together by inserting the protrusions through corresponding apertures in the first sub-frame. When the hybrid frame is in the expanded configuration, the second sub-frame is configured to resist radial compression of the frame.

In a representative embodiment, an implantable prosthetic device comprises a hybrid frame that is radially expandable and compressible between a radially compressed configuration and a radially expanded configuration. The hybrid frame comprising a first sub-frame comprising a plurality of struts pivotably coupled to one another, the first sub-frame comprising one or more expansion and locking mechanisms, and a self-contracting second sub-frame coupled to the first sub-frame and configured to exert a radially inwardly directed contracting force on the first sub-frame.

In another representative embodiment, an implantable prosthetic device comprises a radially compressible and expandable frame. The frame comprises a mechanically expandable first sub-frame comprising a plurality of struts pivotably coupled to one another, and a self-expandable second sub-frame coupled to the first sub-frame, the second sub-frame being axially longer than the first sub-frame and comprising an extending portion that extends axially past the first sub-frame.

In a representative embodiment, a method comprises inserting a distal end of a delivery apparatus into the vasculature of a patient, the delivery apparatus releasably coupled to a prosthetic valve movable between a radially compressed and a radially expanded configuration, the prosthetic valve including a hybrid frame having a mechanically expandable first sub-frame comprising one or more expansion mechanisms, and a self-expanding second sub-frame coupled to the first sub-frame. The method further comprises advancing the prosthetic valve to a selected implantation site, radially expanding the prosthetic valve by actuating the one or more expansion mechanisms to radially expand the first sub-frame and allowing the second sub-frame to self-expand, and allowing the second sub-frame to exert a radially outward force against the first sub-frame to prevent radial compression of the frame.

In another representative embodiment, a method comprises inserting a distal end of a delivery apparatus into the vasculature of a patient, the delivery apparatus releasably coupled to a prosthetic valve movable between a radially compressed and a radially expanded configuration, the prosthetic valve including a hybrid frame having a mechanically expandable first sub-frame comprising one or more expansion and locking mechanisms, and a self-contracting second sub-frame coupled to the first sub-frame. The method further comprises advancing the prosthetic valve to a selected implantation site, radially expanding the prosthetic valve by actuating the one or more expansion and locking mechanisms to radially expand the first sub-frame to the radially expanded configuration, releasing the expansion and locking mechanisms allowing the self-contracting sub-frame to contract the prosthetic valve to a partially contracted configuration, and positioning the partially contracted prosthetic valve at the selected implantation site.

In another representative embodiment, an implantable prosthetic device comprises a hybrid frame movable between a radially compressed configuration and a radially expanded configuration. The hybrid frame comprises a mechanically-expandable first sub-frame comprising a first set of struts pivotably coupled to one another, and a self-expanding second sub-frame comprising a second set of struts, the second sub-frame being coupled to the first sub-frame via one or more fasteners extending through apertures in the first and second sets of struts. The second sub-frame can be configured to lock the hybrid frame in the radially expanded configuration.

In another representative embodiment, an implantable prosthetic device can comprise a hybrid frame movable between a radially compressed configuration and a radially expanded configuration. The hybrid frame comprising a mechanically-expandable first sub-frame comprising a first set of struts pivotably coupled to one another, a plurality of expansion mechanisms coupled to the first sub-frame at circumferentially spaced locations, and a self-expanding second sub-frame comprising a plurality of extension members, each extension member extending between two adjacent expansion mechanisms. When the hybrid frame is in the expanded configuration, the extension members are configured to resist radial compression of the frame.

The foregoing and other objects, features, and advantages will become more apparent from the following detailed description, which proceeds with reference to the accompanying figures.

### BRIEF DESCRIPTION OF THE DRAWINGS

FIG. 1 is a perspective view of a prosthetic heart valve, according to one embodiment.
FIG. 2A is a side elevation view of the frame of the prosthetic heart valve of FIG. 1, shown in a radially compressed state.
FIG. 2B is a side elevation view of the frame of the prosthetic heart valve of FIG. 1, shown in a radially expanded state.
FIG. 3 is a perspective view of a prosthetic valve frame, shown in a radially collapsed state, having a plurality of expansion and locking mechanisms, according to another embodiment.
FIG. 4 is a perspective view of the frame and the expansion and locking mechanisms of FIG. 3, with the frame shown in a radially expanded state.
FIG. 5A is a perspective view of a screw of one of the expansion and locking mechanisms of FIG. 3.
FIG. 5B is a perspective view of one of the expansion and locking mechanisms of FIG. 3_{∘}
FIG. 5C is another perspective view of the frame and the expansion and locking mechanisms of FIG. 3, with the frame shown in a radially expanded state.
FIG. 6 is another perspective view of one of the expansion and locking mechanisms of FIG. 3.
FIG. 7 shows a cross sectional view of one of the expansion and locking mechanisms of FIG. 3 along with a portion of the frame.
FIG. 8 is side elevation view of a delivery apparatus for a prosthetic heart valve, according to one embodiment.
FIG. 9 is a perspective view of a hybrid frame for a prosthetic heart valve having a plurality of expansion mechanisms, according to one embodiment.
FIG. 10 is a side elevational view of the hybrid frame of FIG. 9.
FIG. 11A is a perspective view of a self-expanding sub-frame of a hybrid frame, according to one embodiment.
FIG. 11B is an enlarged portion of the sub-frame of FIG. 11A.
FIG. 12A is a perspective view of a mechanically-expandable sub-frame of a hybrid frame, according to one embodiment.
FIG. 12B is an enlarged portion of the sub-frame of FIG. 12A.
FIG. 13 is a perspective view of a hybrid frame comprising the sub-frames of FIG. 11A and FIG. 12A.
FIG. 14 is a cross-sectional side view of a hybrid frame, according to one embodiment.
FIG. 15 is a cross-sectional side view of a hybrid frame, according to another embodiment.
FIG. 16 is a side elevational view of a self-expanding sub-frame, according to one embodiment.

### DETAILED DESCRIPTION

### General Considerations

For purposes of this description, certain aspects, advantages, and novel features of the embodiments of this disclosure are described herein. The disclosed methods, apparatus, and systems should not be construed as being limiting in any way. Instead, the present disclosure is directed toward all novel and nonobvious features and aspects of the various disclosed embodiments, alone and in various combinations and sub-combinations with one another. The methods, apparatus, and systems are not limited to any specific aspect or feature or combination thereof, nor do the disclosed embodiments require that any one or more specific advantages be present or problems be solved.

Although the operations of some of the disclosed embodiments are described in a particular, sequential order for convenient presentation, it should be understood that this manner of description encompasses rearrangement, unless a particular ordering is required by specific language set forth below. For example, operations described sequentially may in some cases be rearranged or performed concurrently. Moreover, for the sake of simplicity, the attached figures may not show the various ways in which the disclosed methods can be used in conjunction with other methods. Additionally, the description sometimes uses terms like "provide" or "achieve" to describe the disclosed methods. These terms are high-level abstractions of the actual operations that are performed. The actual operations that correspond to these terms may vary depending on the particular implementation and are readily discernible by one of ordinary skill in the art.

All features described herein are independent of one another and, except where structurally impossible, can be used in combination with any other feature described herein. For example, the coupling mechanism of prosthetic valve 500 can be used with prosthetic valves 400, 600, and/or 700.

As used in this application and in the claims, the singular forms "a," "an," and "the" include the plural forms unless the context clearly dictates otherwise. Additionally, the term "includes" means "comprises." Further, the terms "coupled" and "associated" generally mean electrically, electromagnetically, and/or physically (e.g., mechanically or chemically) coupled or linked and does not exclude the presence of intermediate elements between the coupled or associated items absent specific contrary language.

In the context of the present application, the terms "lower" and "upper" are used interchangeably with the terms "inflow" and "outflow", respectively. Thus, for example, the lower end of the valve is its inflow end and the upper end of the valve is its outflow end.

As used herein, the term "proximal" refers to a position, direction, or portion of a device that is closer to the user and further away from the implantation site. As used herein, the term "distal" refers to a position, direction, or portion of a device that is further away from the user and closer to the implantation site. Thus, for example, proximal motion of a device is motion of the device toward the user, while distal motion of the device is motion of the device away from the user. The terms "longitudinal" and "axial" refer to an axis extending in the proximal and distal directions. unless otherwise expressly defined.

### Examples of the Disclosed Technology

Described herein are embodiments of frames for use in prosthetic implants, such as prosthetic valves (e.g., prosthetic heart valves or venous valves), stents, or grafts, to name a few. The frames can comprise one or more sub-frames which can be mechanically expandable and/or self-expanding.

Prosthetic valves disclosed herein can be radially compressible and expandable between a radially compressed state and a radially expanded state. Thus, the prosthetic valves can be crimped on or retained by an implant delivery apparatus in the radially compressed state during delivery, and then expanded to the radially expanded state once the prosthetic valve reaches the implantation site. It is understood that the valves disclosed herein may be used with a variety of implant delivery apparatuses, and examples thereof will be discussed in more detail later.

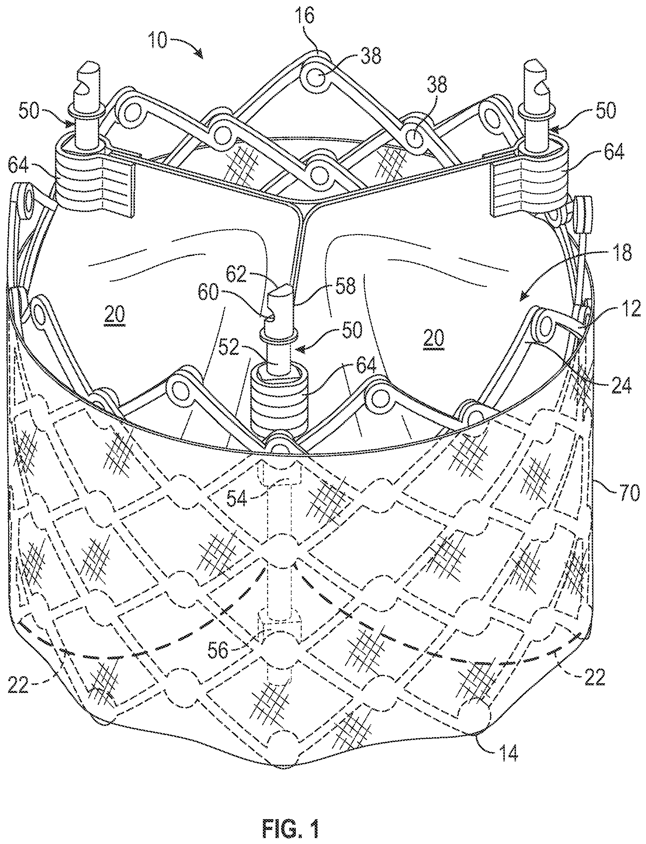

FIG. 1 shows an exemplary mechanically-expandable prosthetic valve 10, according to one embodiment. The prosthetic valve 10 can include an annular stent or frame 12 having an inflow end 14 and an outflow end 16. The prosthetic valve 10 can also include a valvular structure 18 which is coupled to and supported inside of the frame 12. The valvular structure 18 is configured to regulate the flow of blood through the prosthetic valve 10 from the inflow end 14 to the outflow end 16.

The valvular structure 18 can include, for example, a leaflet assembly comprising one or more leaflets 20 made of a flexible material. The leaflets 20 can be made from in whole or part, biological material, bio-compatible synthetic materials, or other such materials. Suitable biological material can include, for example, bovine pericardium (or pericardium from other sources). The leaflets 20 can be secured to one another at their adjacent sides to form commissures, each of which can be secured to a respective actuator 50 or the frame 102.

In the depicted embodiment, the valvular structure 18 comprises three leaflets 20, which can be arranged to collapse in a tricuspid arrangement. Each leaflet 20 can have an inflow edge portion 22. As shown in FIG. 1, the inflow edge portions 22 of the leaflets 20 can define an undulating, curved scallop shape that follows or tracks a plurality of interconnected strut segments of the frame 12 in a circumferential direction when the frame 12 is in the radially expanded configuration. The inflow edges of the leaflets can be referred to as a "scallop line."

In some embodiments, the inflow edge portions 22 of the leaflets 20 can be sutured to adjacent struts of the frame generally along the scallop line. In other embodiments, the inflow edge portions 22 of the leaflets 20 can be sutured to an inner skirt, which in turn in sutured to adjacent struts of the frame. By forming the leaflets 20 with this scallop geometry, stresses on the leaflets 20 are reduced, which in turn improves durability of the valve 10. Moreover, by virtue of the scallop shape, folds and ripples at the belly of each leaflet 20 (the central region of each leaflet), which can cause early calcification in those areas, can be eliminated or at least minimized. The scallop geometry also reduces the amount of tissue material used to form valvular structure 18, thereby allowing a smaller, more even crimped profile at the inflow end 14 of the valve 10.

Further details regarding transcatheter prosthetic heart valves, including the manner in which the valvular structure can be mounted to the frame of the prosthetic valve can be found, for example, in U.S. Patent Nos. 6,730,118, 7,393,360, 7,510,575, 7,993,394, and 8,252,202, U.S. Publication Nos. 2018/0325665 and 2020/0352711.

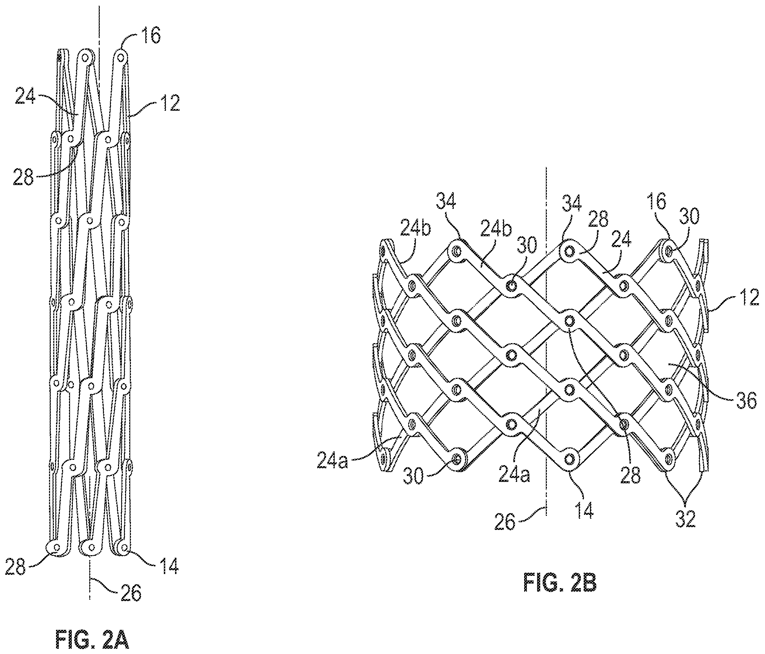

The prosthetic valve 10 can be radially compressible and expandable between a radially compressed configuration and a radially expanded configuration. FIGS. 2A-2B show the bare frame 12 of the prosthetic valve 10 (without the leaflets and other components) for purposes of illustrating expansion of the prosthetic valve 10 from the radially compressed configuration (FIG. 2A) to the radially expanded configuration (FIG. 2B).

The frame 12 can include a plurality of interconnected lattice struts 24 arranged in a lattice-type pattern and forming a plurality of apices 34 at the outflow end 16 of the prosthetic valve 10. The struts 24 can also form similar apices 32 at the inflow end 14 of the prosthetic valve 10. In FIG. 2B, the struts 24 are shown as positioned diagonally, or offset at an angle relative to, and radially offset from, a longitudinal axis 26 of the prosthetic valve 10 when the prosthetic valve 10 is in the expanded configuration. In other implementations, the struts 24 can be offset by a different amount than depicted in FIG. 2B, or some or all of the struts 24 can be positioned parallel to the longitudinal axis 26 of the prosthetic valve 10.

The struts 24 can comprise a set of inner struts 24a (extending from the lower left to the upper right of the frame in FIG. 2B) and a set of outer struts 24b (extending from the upper left to the lower right of the frame in FIG. 2B) connected to the inner struts 24a. The open lattice structure of the frame 12 can define a plurality of open frame cells 36 between the struts 24.

The struts 24 can be pivotably coupled to one another at one or more pivot joints or pivot junctions 28 along the length of each strut. For example, in one embodiment, each of the struts 24 can be formed with apertures 30 at opposing ends of the strut and apertures spaced along the length of the strut. Respective hinges can be formed at the locations where struts 24 overlap each other via fasteners 38 (FIG. 1), such as rivets or pins that extend through the apertures 30. The hinges can allow the struts 24 to pivot relative to one another as the frame 12 is radially expanded or compressed, such as during assembly, preparation, or implantation of the prosthetic valve 10.

The frame struts and the components used to form the pivot joints of the frame 12 (or any frames described below) can be made of any of various suitable materials, such as stainless steel, a cobalt chromium alloy, or a nickel titanium alloy ("NiTi"), for example Nitinol. In some embodiments, the frame 12 can be constructed by forming individual components (e.g., the struts and fasteners of the frame) and then mechanically assembling and connecting the individual components together. Further details regarding the construction of the frame and the prosthetic valve are described in U.S. Patent No. 10,603,165 and 10,869,759 and U.S. Patent Publication Nos. 2019/0060057 and 2020/0188099.

In the illustrated embodiment, the prosthetic valve 10 can be mechanically expanded from the radially contracted configuration to the radially expanded configuration. For example, the prosthetic valve 10 can be radially expanded by maintaining the inflow end 14 of the frame 12 at a fixed position while applying a force in the axial direction against the outflow end 16 toward the inflow end 14. Alternatively, the prosthetic valve 10 can be expanded by applying an axial force against the inflow end 14 while maintaining the outflow end 16 at a fixed position, or by applying opposing axial forces to the inflow and outflow ends 14, 16, respectively.

As shown in FIG. 1, the prosthetic valve 10 can include one or more actuators 50 mounted to and equally spaced around the inner surface of the frame 12. Each of the actuators 50 can be configured to form a releasable connection with one or more respective actuators of a delivery apparatus.

In the illustrated embodiment, expansion and compression forces can be applied to the frame by the actuators 50. Referring again to FIG. 1, each of the actuators 50 can comprise a screw or threaded rod 52, a first anchor in the form of a cylinder or sleeve 54, and a second anchor in the form of a threaded nut 56. The rod 52 extends through the sleeve 54 and the nut 56. The sleeve 54 can be secured to the frame 12, such as with a fastener 38 that forms a hinge at the junction between two struts. Each actuator 50 is configured to increase the distance between the attachment locations of a respective sleeve 54 and nut 56, which causes the frame 12 to elongate axially and compress radially, and to decrease the distance between the attachment locations of a respective sleeve 54 and nut 56, which causes the frame 12 to foreshorten axially and expand radially.

For example, each rod 52 can have external threads that engage internal threads of the nut 56 such that rotation of the rod causes corresponding axial movement of the nut 56 toward or away from the sleeve 54 (depending on the direction of rotation of the rod 52). This causes the hinges supporting the sleeve 54 and the nut 56 to move closer towards each other to radially expand the frame or to move farther away from each other to radially compress the frame, depending on the direction of rotation of the rod 52.

In other embodiments, the actuators 50 can be reciprocating type actuators configured to apply axial directed forces to the frame to produce radial expansion and compression of the frame. For example, the rod 52 of each actuator can be fixed axially relative to the sleeve 56 and slidable relative to the sleeve 54. Thus, in this manner, moving the rod 52 distally relative to the sleeve 54 and/or moving the sleeve 54 proximally relative to the rod 52 radially compresses the frame. Conversely, moving the rod 52 proximally relative to the sleeve 54 and/or moving the sleeve 54 distally relative to the rod 52 radially expands the frame.

When reciprocating type actuators are used, the prosthetic valve can also include one or more locking mechanisms that retain the frame in the expanded state. The locking mechanisms can be separate components that are mounted on the frame apart from the actuators, or they can be a sub-component of the actuators themselves.

Each rod 52 can include an attachment member 58 along a proximal end portion of the rod 52 configured to form a releasable connection with a corresponding actuator of a delivery apparatus. The actuator(s) of the delivery apparatus can apply forces to the rods for radially compressing or expanding the prosthetic valve 10. The attachment member 58 in the illustrated configuration comprises a notch 60 and a projection 62 that can engage a corresponding projection of an actuator of the delivery apparatus.

In the illustrated embodiments, the prosthetic valve 10 includes three such actuators 50, although a greater or fewer number of actuators could be used in other embodiments. The leaflets 20 can have commissure attachments members 64 that wrap around the sleeves 54 of the actuators 50. Further details of the actuators, locking mechanisms and delivery apparatuses for actuating the actuators can be found in U.S. Patent No. 10,603,165 and 10,806,573, and U.S. Patent Publication No. 2018/032566. of the actuators and locking mechanisms disclosed in the previously filed applications can be incorporated in any of the prosthetic valves disclosed herein. Further, any of the delivery apparatuses disclosed in the previously filed applications can be used to deliver and implant any of the prosthetic valves discloses herein.

The prosthetic valve 10 can include one or more skirts or sealing members. In some embodiments, the prosthetic valve 10 can include an inner skirt (not shown) mounted on the inner surface of the frame. The inner skirt can function as a sealing member to prevent or decrease perivalvular leakage, to anchor the leaflets to the frame, and/or to protect the leaflets against damage caused by contact with the frame during crimping and during working cycles of the prosthetic valve. As shown in FIG. 1, the prosthetic valve 10 can also include an outer skirt 70 mounted on the outer surface of the frame 12. The outer skirt 70 can function as a sealing member for the prosthetic valve by sealing against the tissue of the native valve annulus and helping to reduce paravalvular leakage past the prosthetic valve. The inner and outer skirts can be formed from any of various suitable biocompatible materials, including any of various synthetic materials, including fabrics (e.g., polyethylene terephthalate fabric) or natural tissue (e.g., pericardial tissue). Further details regarding the use of skirts or sealing members in prosthetic valve can be found, for example, in U.S. Patent Publication No. 2020/0352711.

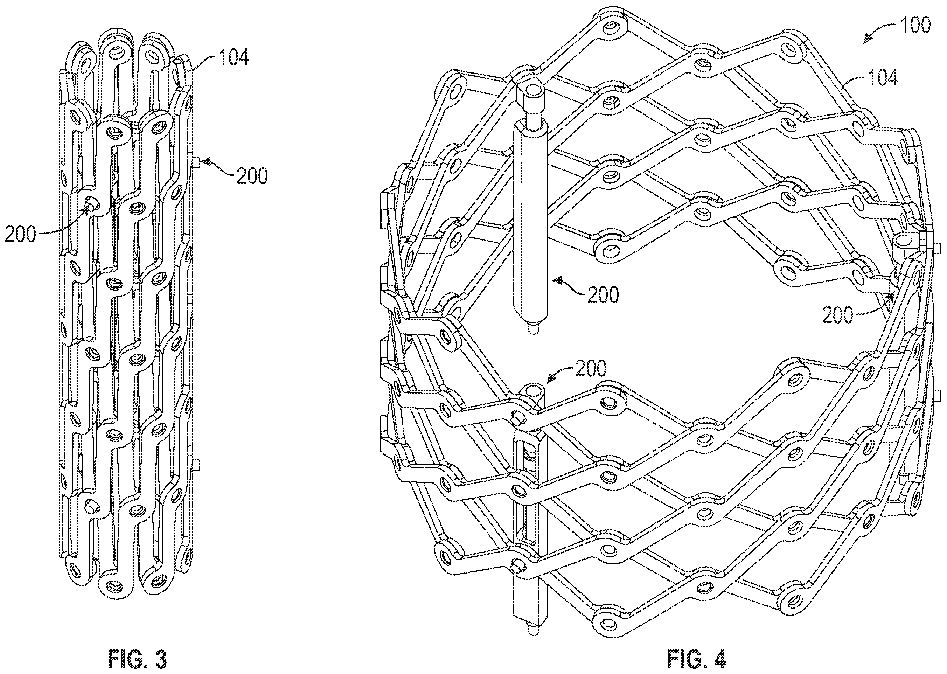

FIGS. 3-4 show another embodiment of a prosthetic valve 100 comprising a frame 104 and expansion and locking mechanisms 200 (also referred to as "actuators"). It should be understood that the prosthetic valve 100 can include leaflets 20 and other soft components, such as one or more skirts 70, which are removed for purposes of illustration. Expansion and locking mechanism 200 can be used to both radially expand and lock the prosthetic valve in a radially expanded state. In the example of FIGS. 3 and 4, three expansion and locking mechanisms 200 are attached to the frame 104 but in other example delivery assemblies, any number of expansion and locking mechanisms 200 can be used. FIG. 3 shows the expansion and locking mechanisms 200 attached to the frame 104 when the frame is in a radially collapsed configuration and FIG. 4 shows expansion and locking mechanisms attached to the frame when the frame is in a radially expanded configuration.

It will be appreciated that prosthetic valve 100 can, in certain embodiments, use other mechanisms for expansion and locking, such as linear actuators, alternate locking mechanisms, and alternate expansion and locking mechanisms. Further details regarding the use of linear actuators, locking mechanisms, and expansion and locking mechanisms in prosthetic valve can be found, for example, in U.S. Patent No. 10,603,165.

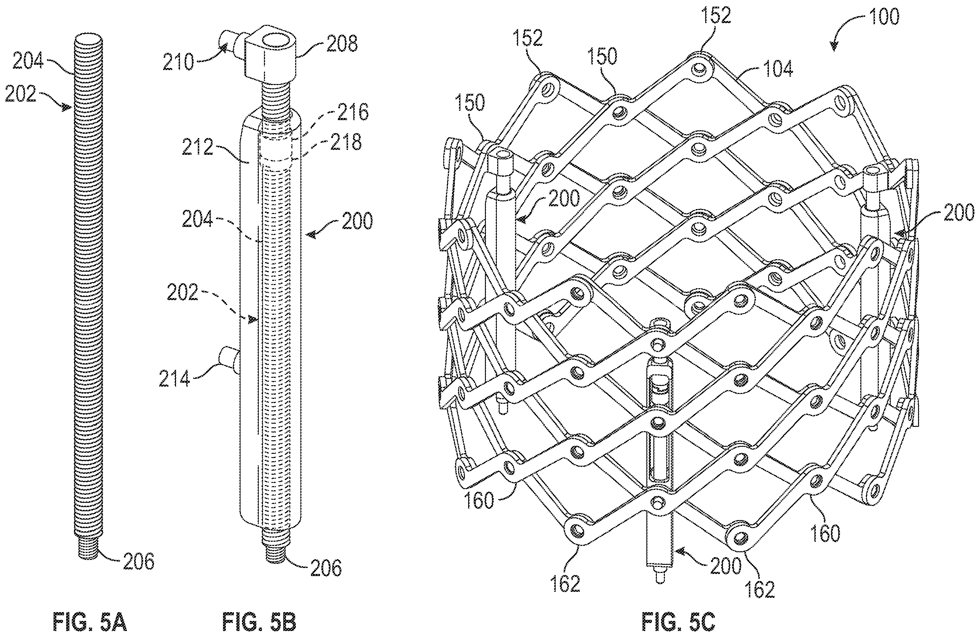

Referring to FIGS. 5A-5C, the expansion and locking mechanism 200 in the illustrated embodiment can include an actuator screw 202 (which functions as a linear actuator or a push-pull member in the illustrated embodiment) comprising a relatively long upper, or distal, portion 204 and a relatively shorter lower, or proximal, portion 206 at the proximal end of the screw 200, wherein the lower portion has a smaller diameter than the upper portion. Both the upper and lower portions 204, 206 of the screw 202 can have externally threaded surfaces.

The actuator screw 200 can have a distal attachment piece 208 attached to its distal end having a radially extending distal valve connector 210. The distal attachment piece 208 can be fixed to the screw 202 (e.g., welded together or manufactured as one piece). The distal valve connector 210 can extend through an opening at or near the distal end of the frame 104 formed at a location on the frame where two or more struts intersect as shown in FIG. 5C. The distal valve connector 210 can be fixed to the frame 104 (e.g., welded). Due to the shape of the struts, the distal end of the frame 104 comprises an alternating series of distal junctions 150 and distal apices 152. In the illustrated example, the distal valve connectors 210 of the three expansion and locking mechanisms 200 are connected to the frame 104 through distal junctions 150. In other examples, one or more distal valve connectors 210 can be connected to the frame 104 through distal apices 152. In other embodiments, the distal valve connectors 210 can be connected to junctions closer to the proximal end of the frame 104.

The expansion and locking mechanism 200 can further include a sleeve 212. The sleeve 212 can be positioned annularly around the distal portion 204 of the screw 202 and can contain axial openings at its proximal and distal ends through which the screw 202 can extend. The axial openings and the lumen in the sleeve 212 can have a diameter larger than the diameter of the distal portion 204 of the screw 202 such that the screw can move freely within the sleeve (the screw 202 can be moved proximally and distally relative to the sleeve 212). Because the actuator screw 202 can move freely within the sleeve, it can be used to radially expand and/or contract the frame 104 as disclosed in further detail below.

The sleeve 212 can have a proximal valve connector 214 extending radially from its outer surface. The proximal valve connector 214 can be fixed to the sleeve 212 (e.g., welded). The proximal valve connector 214 can be axially spaced from the distal valve connector 210 such that the proximal valve connector can extend through an opening at or near the proximal end of the frame 104. The proximal end of the frame 104 comprises an alternating series of proximal junctions 160 and proximal apices 162. In the illustrated example, the proximal valve connectors 214 of the three expansion and locking mechanisms 200 are connected to the frame 104 through proximal junctions 160. In other examples, one or more proximal valve connectors 214 can be connected to the frame 104 through proximal apices 162. In other embodiments, the proximal valve connectors 214 can be connected to junctions closer to the distal end of the frame 104.

It should be understood that the distal and proximal connectors 210, 214 need not be connected to opposite ends of the frame. The actuator 200 can be used to expand and compress the frame as long as the distal and proximal connectors are connected to respective junctions on the frame that are axially spaced from each other.

A locking nut 216 can be positioned inside of the sleeve 212 and can have an internally threaded surface that can engage the externally threaded surface of the actuator screw 202. The locking nut 216 can have a notched portion 218 at its proximal end, the purpose of which is described below. The locking nut can be used to lock the frame 104 into a particularly radially expanded state, as discussed below.

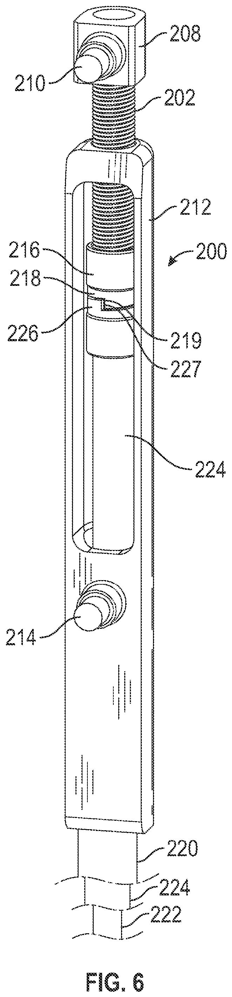

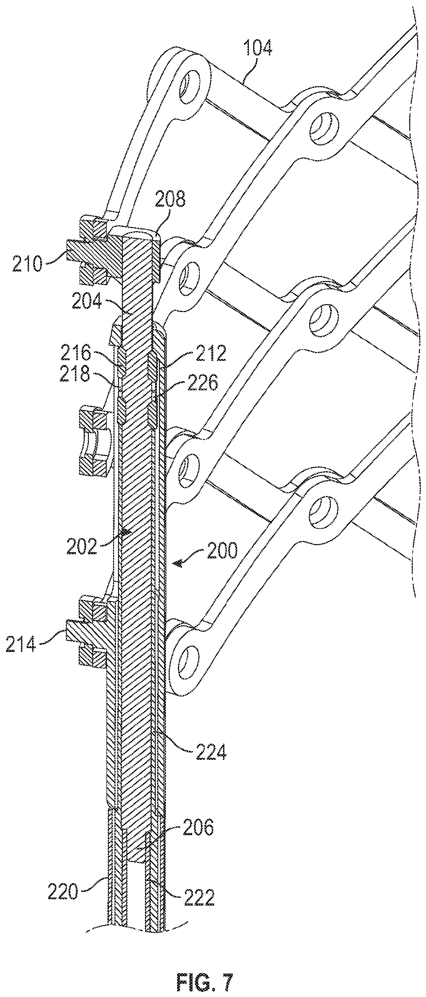

FIGS. 6 and 7 shows the expansion and locking mechanism 200 including components of a delivery apparatus not shown in FIGS. 5A-5C. As shown, the expansion and locking mechanism 200 can be releasably coupled to a support tube 220, an actuator member 222, and a locking tool 224. The proximal end of the support tube 220 can be connected to a handle or other control device (not shown) that a doctor or operator of the delivery assembly utilizes to operate the expansion and locking mechanism 200 as described herein. Similarly, the proximal ends of the actuator member 222 and the locking tool 224 can be connected to the handle.

The support tube 220 annularly surrounds a proximal portion of the locking tool 224 such that the locking tool extends through a lumen of the support tube. The support tube 220 and the sleeve are sized such that the distal end of the support tube abuts or engages the proximal end of the sleeve 212 such that the support tube is prevented from moving distally beyond the sleeve.

The actuator member 222 extends through a lumen of the locking tool 224. The actuator member 222 can be, for example, a shaft, a rod, a cable, or wire. The distal end portion of the actuator member 222 can be releasably connected to the proximal end portion 206 of the actuator screw 202. For example, the distal end portion of the actuator member 222 can have an internally threaded surface that can engage the external threads of the proximal end portion 206 of the actuator screw 202. Alternatively, the actuator member 222 can have external threads that engage an internally threaded portion of the screw 202. When the actuator member 222 is threaded onto the actuator screw 202, axial movement of the actuator member causes axial movement of the screw.

The distal portion of the locking tool 224 annularly surrounds the actuator screw 202 and extends through a lumen of the sleeve 212 and the proximal portion of the locking tool annularly surrounds the actuator member 222 and extends through a lumen of the support tube 220 to the handle of the delivery device. The locking tool 224 can have an internally threaded surface that can engage the externally threaded surface of the locking screw 202 such that clockwise or counter-clockwise rotation of the locking tool 224 causes the locking tool to advance distally or proximally along the screw, respectively.

The distal end of the locking tool 224 can comprise a notched portion 226, as can best be seen in FIG. 6. The notched portion 226 of the locking tool 224 can have an engagement surface 227 that is configured to engage a correspondingly shaped engagement surface 219 of the notched portion 218 of the locking nut 216 such that rotation of the locking tool (e.g., clockwise rotation) causes the nut 216 to rotate in the same direction (e.g., clockwise) and advance distally along the locking screw 202. The notched portions 218, 226 in the illustrated embodiment are configured such that rotation of the locking tool 224 in the opposite direction (e.g., counter-clockwise) allows the notched portion 226 of the tool 224 to disengage the notched portion 218 of the locking nut 216; that is, rotation of the locking tool in a direction that causes the locking tool to move proximally does not cause corresponding rotation of the nut.

In alternative embodiments, the distal end portion of the locking tool 224 can have various other configurations adapted to engage the nut 216 and produce rotation of the nut upon rotation of the locking tool for moving the nut distally, such as any of the tool configurations described herein. In some embodiments, the distal end portion of the locking tool 224 can be adapted to produce rotation of the nut 216 in both directions so as move the nut distally and proximally along the locking screw 202.

In operation, prior to implantation, the actuator member 222 is screwed onto the proximal end portion 206 of the actuator screw 202 and the locking nut 216 is rotated such that it is positioned at the proximal end of the screw. The frame 104 can then be placed in a radially collapsed state and the delivery assembly can be inserted into a patient. Once the prosthetic valve is at a desired implantation site, the frame 104 can be radially expanded as described herein.

To radially expand the frame 104, the support tube 220 is held firmly against the sleeve 212. The actuator member 222 is then pulled in a proximal direction through the support tube, such as by pulling on the proximal end of the actuator member or actuating a control knob on the handle that produces proximal movement of the actuator member. Because the support tube 220 is being held against the sleeve 212, which is connected to a proximal end of the frame 104 by the proximal valve connector 214, the proximal end of the frame is prevented from moving relative to the support tube. As such, movement of the actuator member 222 in a proximal direction causes movement of the actuator screw 202 in a proximal direction (because the actuator member is threaded onto the screw), thereby causing the frame 104 to foreshorten axially and expand radially. Alternatively, the frame 104 can be expanded by moving the support tube 220 distally while holding the actuator member 222 stationary or moving the support tube distally while moving the actuator member 222 proximally.

After the frame 104 is expanded to a desired radially expanded size, the frame can be locked at this radially expanded size as described herein. Locking the frame can be achieved by rotating the locking tool 224 in a clockwise direction causing the notched portion 226 of the locking tool to engage the notched portion 218 of the locking nut 216, thereby advancing the locking nut distally along the actuator screw 202. The locking tool 224 can be so rotated until the locking nut 216 abuts an internal shoulder at the distal end of the sleeve 212 and the locking nut 216 cannot advance distally any further (see FIG. 6). This will prevent the screw 202 from advancing distally relative to the sleeve 212 and radially compressing the frame 104. However, in the illustrated embodiment, the nut 216 and the screw 202 can still move proximally through the sleeve 212, thereby allowing additional expansion of the frame 104 either during implantation or later during a valve-in-valve procedure.

Once the frame 104 is locked in radially expanded state, the locking tool 224 can be rotated in a direction to move the locking tool proximally (e.g., in a counter-clockwise direction) to decouple the notched portion 226 from the notched portion 218 of the locking nut 216 and to unscrew the locking tool from the actuator screw 204. Additionally, the actuator member 222 can be rotated in a direction to unscrew the actuator member from the lower portion 206 of the actuator screw 202 (e.g., the actuator member 222 can be configured to disengage from the actuator screw when rotated counter-clockwise). Once the locking tool 224 and the actuator member 222 are unscrewed from the actuator screw 204, they can be removed from the patient along with the support tube 220, leaving the actuator screw and the sleeve 212 connected to the frame 104, as shown in FIG. 5C, with the frame 104 locked in a particular radially-expanded state.

In an alternative embodiment, the locking tool 224 can be formed without internal threads that engage the external threads of the actuator screw 202, which can allow the locking tool 224 to be slid distally and proximally through the sleeve 212 and along the actuator screw 202 to engage and disengage the nut 216.

In some embodiments, additional designs for expansion and locking mechanisms can be used instead of the design previously described. Details on expansion and locking mechanisms can be found, for example, in U.S. Patent No. 10,603,165.

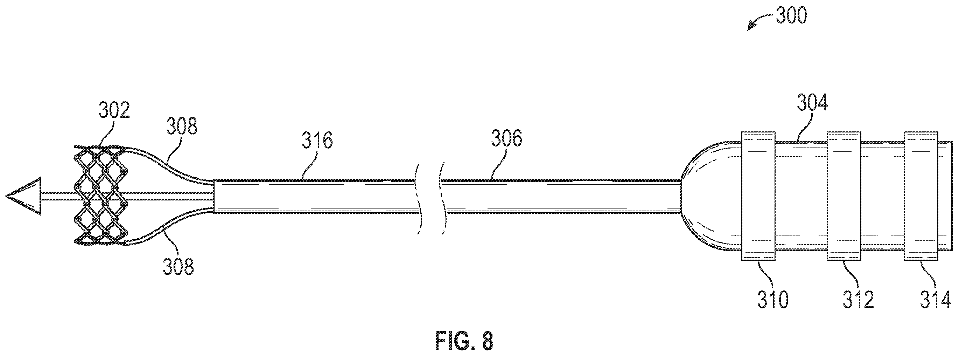

FIG. 8 illustrates a delivery apparatus 300, according to one embodiment, adapted to deliver a prosthetic heart valve 302, such as the illustrated prosthetic heart valve 10 or 100, described above. The prosthetic valve 302 can be releasably coupled to the delivery apparatus 300. It should be understood that the delivery apparatus 300 and other delivery apparatuses disclosed herein can be used to implant prosthetic devices other than prosthetic valves, such as stents or grafts.

The delivery apparatus 300 in the illustrated embodiment generally includes a handle 304, a first elongated shaft 306 (which comprises an outer shaft in the illustrated embodiment) extending distally from the handle 304, at least one actuator assembly 308 extending distally through the outer shaft 306. The at least one actuator assembly 308 can be configured to radially expand and/or radially collapse the prosthetic valve 302 when actuated.

Though the illustrated embodiment shows two actuator assemblies 308 for purposes of illustration, it should be understood that one actuator 308 can be provided for each actuator on the prosthetic valve. For example, three actuator assemblies 308 can be provided for a prosthetic valve having three actuators. In other embodiments, a greater or fewer number of actuator assemblies can be present.

In some embodiments, a distal end portion 316 of the shaft 306 can be sized to house the prosthetic valve in its radially compressed, delivery state during delivery of the prosthetic valve through the patient's vasculature. In this manner, the distal end portion 316 functions as a delivery sheath or capsule for the prosthetic valve during delivery,

The actuator assemblies 308 can be releasably coupled to the prosthetic valve 302. For example, in the illustrated embodiment, each actuator assembly 308 can be coupled to a respective actuator 200 of the prosthetic valve 302. Each actuator assembly 308 can comprise a support tube 220, an actuator member 222, and a locking tool 224. When actuated, the actuator assembly can transmit pushing and/or pulling forces to portions of the prosthetic valve to radially expand and collapse the prosthetic valve as previously described. The actuator assemblies 308 can be at least partially disposed radially within, and extend axially through, one or more lumens of the outer shaft 306. For example, the actuator assemblies 308 can extend through a central lumen of the shaft 306 or through separate respective lumens formed in the shaft 306.

The handle 302 of the delivery apparatus 300 can include one or more control mechanisms (e.g., knobs or other actuating mechanisms) for controlling different components of the delivery apparatus 300 in order to expand and/or deploy the prosthetic valve 10. For example, in the illustrated embodiment the handle 302 comprises first, second, and third knobs 310, 312, and 314.

The first knob 310 can be a rotatable knob configured to produce axial movement of the outer shaft 306 relative to the prosthetic valve 302 in the distal and/or proximal directions in order to deploy the prosthetic valve from the delivery sheath 316 once the prosthetic valve has been advanced to a location at or adjacent the desired implantation location with the patient's body. For example, rotation of the first knob 310 in a first direction (e.g., clockwise) can retract the sheath 316 proximally relative to the prosthetic valve 302 and rotation of the first knob 310 in a second direction (e.g., counter-clockwise) can advance the sheath 316 distally. In other embodiments, the first knob 310 can be actuated by sliding or moving the knob 310 axially, such as pulling and/or pushing the knob. In other embodiments, actuation of the first knob 310 (rotation or sliding movement of the knob 310) can produce axial movement of the actuator assemblies 308 (and therefore the prosthetic valve 302) relative to the delivery sheath 316 to advance the prosthetic valve distally from the sheath 316.

The second knob 312 can be a rotatable knob configured to produce radial expansion and/or contraction of the prosthetic valve 302. For example, rotation of the second knob 312 can move the actuator member 222 and the support tube 220 axially relative to one another. Rotation of the second knob 312 in a first direction (e.g., clockwise) can radially expand the prosthetic valve 302 and rotation of the second knob 312 in a second direction (e.g., counter-clockwise) can radially collapse the prosthetic valve 302. In other embodiments, the second knob 312 can be actuated by sliding or moving the knob 312 axially, such as pulling and/or pushing the knob.

The third knob 314 can be a rotatable knob configured to retain the prosthetic heart valve 302 in its expanded configuration. For example, the third knob 314 can be operatively connected to a proximal end portion of the locking tool 224 of each actuator assembly 308. Rotation of the third knob in a first direction (e.g., clockwise) can rotate each locking tool 224 to advance the locking nuts 216 to their distal positions to resist radial compression of the frame of the prosthetic valve, as described above. Rotation of the knob 314 in the opposite direction (e.g., counterclockwise) can rotate each locking tool 224 in the opposite direction to decouple each locking tool 224 from the respective nut 216 and remove the locking tool 224 from the respective actuator screw 202. In other embodiments, the third knob 314 can be actuated by sliding or moving the third knob 314 axially, such as pulling and/or pushing the knob.

Although not shown, the handle 304 can include a fourth rotatable knob operative connected to a proximal end portion of each actuator member 222. The fourth knob can be configured to rotate each actuator member 222, upon rotation of the knob, to unscrew each actuator member 222 from the proximal portion 206 of a respective actuator 202. As described above, once the locking tools 224 and the actuator members 222 are unscrewed form the actuator screws 204, they can be removed from the patient along with the support tubes 220.

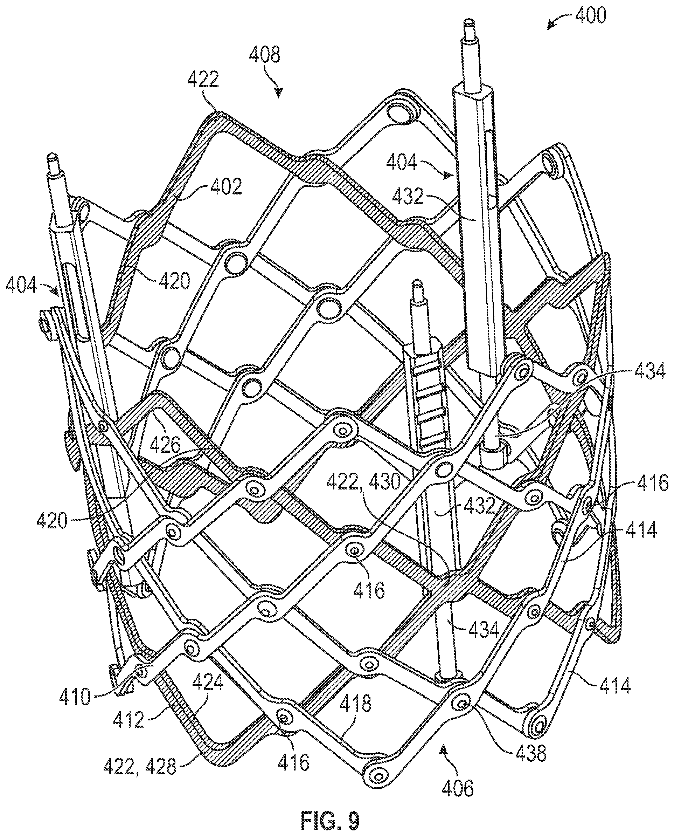

FIGS. 9-10 illustrate an exemplary embodiment of a prosthetic heart valve 400 comprising a hybrid frame 402 and one or more expansion mechanisms 404. The expansion mechanisms 404 can be used to radially expand the frame 402 as described in more detail below.

The prosthetic valve 400 can include a valvular structure (e.g., valvular structure 18) and inner and/or outer skirts, as previously described, although these components are omitted for purposes of illustration. The frame 402 can comprise an inflow end portion 406 (which is the distal end of the frame in the delivery configuration for the illustrated embodiment), and an outflow end portion 408 (which is the proximal end of the frame in the delivery configuration for the illustrated embodiment).

The frame 402 can include a plurality of sub-frames coupled to one another to create a hybrid frame. In the illustrated embodiment, the hybrid frame 402 can comprise a first sub-frame 410 and a second sub-frame 412. FIG. 9 shows the assembled hybrid frame 402 with stippling added to the struts of the second sub-frame 412 for purposes of illustration. The stippling is added to distinguish the first sub-frame 410 from the second sub-frame 412 and does not represent actual surface ornamentation. In other embodiments, the hybrid frame 402 can comprise any number of sub-frames, for example, one sub-frame, three sub-frames, or four sub-frames.

The first sub-frame 410 of the hybrid frame 402 can be a mechanically-expandable sub-frame comprising a plurality of pivotably connected struts 414 arranged in a lattice-type pattern. Each strut 414 can fully extend from the inflow end portion 406 of the frame 402 to the outflow end portion 408. Thus, in the illustrated embodiment, the first sub-frame 410 can be formed entirely from struts 414 that extend continuously from the inflow end portion to the outflow end portion. In alternative embodiments, the first sub-frame 410 can have struts that are connected end-to-end along the length of the sub-frame 410.

Each strut 414 of the first sub-frame 410 can comprise a plurality of apertures used to pivotably connect the struts 414 to one another. Respective hinges can be formed at the locations where the struts 414 overlap each other via fasteners 416, such as rivets or pins that extend through the apertures. The hinges can allow the struts 414 to pivot relative to one another as the first sub-frame 410 is radially expanded or compressed, such as during assembly, preparation, or implantation of the prosthetic valve 400. The struts 414 can define one or more cells 418. In some embodiments, one or more portions of the cell 418 can be defined by the second sub-frame 412. In the illustrated embodiment, the hybrid frame 402 can comprise five rows of cells 418. However, in other embodiments, the first sub-frame can comprise a greater or fewer number of cells 418.

The first sub-frame 410 can comprise one or more expansion mechanisms 404, as described in more detail below. The expansion mechanisms 404 can be coupled to the first sub-frame 410 and can be configured to move the first sub-frame 410 (and therefore the hybrid frame 402) between a radially compressed configuration and a radially expanded configuration.

The second sub-frame 412 can be a self-expanding sub-frame, that is, the sub-frame 412 can be biased such that it can radially expand to a functional size in the absence of any expansion mechanisms and can remain at a functional size in the absence of any locking mechanisms. Due to its self-expanding configuration, the second sub-frame 412 can exert a radially-outwardly directed force (e.g., away from a central longitudinal axis of the hybrid frame 402), that can be used to secure the prosthetic heart valve 400 at the selected implantation site and resist compression of the hybrid frame 402. The second sub-frame 412 can be configured to retain the prosthetic valve 400 in the expanded configuration against compressive radial forces applied to the prosthetic valve by the patient's anatomy (e.g., the native aortic annulus).

As shown in the illustrated embodiment, the second sub-frame 412 can comprise a plurality of longitudinally-extending frame members or struts 420. In some embodiments, the struts 420 can be formed from a unitary piece of material. In other embodiments, the struts 420 can be welded or otherwise secured together at junctions 422. In particular embodiments, the second sub-frame 412 can be laser cut from a metal tube (e.g., a Nitinol tube) wherein the laser cutting forms the struts 420. The struts 420 can be made of a suitable shape memory material, such as Nitinol (nickel titanium alloy), that allows for the second sub-frame to be compressed to a reduced diameter for delivery in a delivery apparatus (such as delivery apparatus 300, described above) and then causes the second sub-frame 412 to radially expand and remain at its functional size inside the patient's body when deployed from the delivery apparatus.

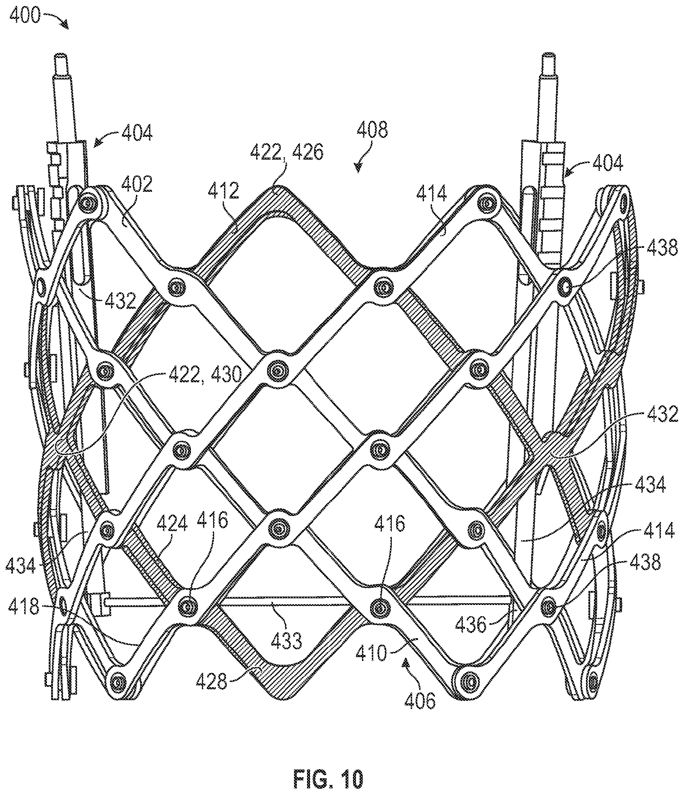

As best seen in FIG. 10, the struts 420 can define one or more cells 424, each cell having an inflow apex 426, an outflow apex 428, and two side junctions 430. In the illustrated embodiment, the second sub-frame 412 comprises three cells 424 arranged circumferentially around the hybrid frame 402. However, in other embodiments, the second sub-frame 412 can comprise a greater or fewer number of cells 424.

The first and second sub-frames 410, 412 can be coupled together such that one frame is positioned radially inwardly of the other frame. For example, in the illustrated embodiment, the first sub-frame 410 is positioned radially outwardly of the second sub-frame 412, such that the first sub-frame 410 is an outer sub-frame and the second sub-frame 412 is an inner sub-frame. In other embodiments, the second sub-frame 412 can be the outer sub-frame and the first sub-frame 410 can be the inner sub-frame. In still other embodiments, the first and second sub-frames 410, 412 can be coupled together in a woven or latticed manner such that portions of the second sub-frame 412 are positioned radially outwardly of the first sub-frame 410 and vice versa.

The first and second sub-frames 410, 412 can be coupled to one another using any of a variety of methods. For example, the sub-frames 410, 412 can be coupled together using fasteners such as rivets or pins, or by other means of attachment such as sutures, welding or adhesives. For example, in the illustrated embodiment, the first and second sub-frames are coupled together via one or more fasteners 416 extending through apertures in struts of the first and second sub-frames 410, 412 at junctions where the struts overlap each other.

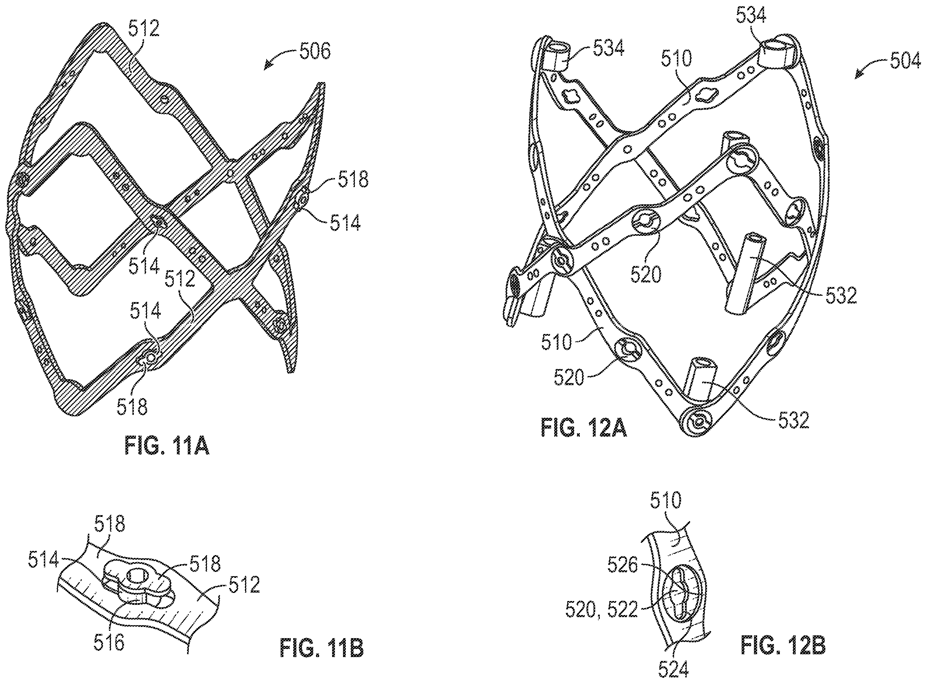

In other embodiments, the first and second sub-frames 410, 412 can be coupled together using one or more expansion mechanisms 404, as described in more detail below. In still other embodiments, the sub-frames 410, 412 can be coupled together using a plurality of interlocking engagement members and apertures, as described in more detail below with reference to FIGS. 11-13.

In embodiments wherein the second sub-frame 412 is the inner frame, the valvular structure can be coupled to the second sub-frame 412. Coupling the valvular structure to the self-expanding Nitinol sub-frame 412 can advantageously improve hemodynamic flow through the prosthetic valve. For example, the flexibility of the Nitinol frame can help improve the hemodynamics of the prosthetic valve 400 by acting as a force dampener and thereby reducing the force load on the leaflets.

In some particular embodiments, the self-expanding second sub-frame 412 may not exert a sufficient radial expansion force to fully expand the mechanically-expandable first sub-frame 410. Accordingly, the expansion mechanisms 404 can be used to move the first sub-frame 410 between the radially compressed configuration and the radially expanded configuration. For example, the expansion mechanisms 404 can exert a force of more than about 100 N to expand the first sub-frame 410. However, the second sub-frame 412 can generate sufficient force to retain the hybrid frame 402 in the expanded configuration against the forces exerted by the native anatomy. For example, the second sub-frame 412 can exert an outward radial force of between about 20 N and about 40 N to retain the prosthetic valve in the expanded configuration.

In some alternative embodiments, in lieu of or in addition to the second sub-frame 412, the prosthetic valve 400 can comprise one or more extension members 433 (FIG. 10). The extension members 433 can be coupled to and extend between one or more sets of adjacent expansion mechanisms 404. The extension members 433 can be formed from a self-expanding material (such as Nitinol). When the prosthetic heart valve 400 is in the expanded configuration, the extension members 433 can extend substantially perpendicular to the longitudinal axis of the prosthetic valve 400. When the prosthetic valve 400 is in the radially compressed configuration, the extension members 433 can deform axially toward the inflow and/or outflow portions 406, 408 of the prosthetic valve 400.

As the first sub-frame 410 is expanded using the expansion mechanisms 404, the extension members can expand into an expanded configuration designed to retain the prosthetic valve 400 at its functional size inside the patient's body against compressive radial forces applied to the prosthetic valve by the patient's anatomy (e.g., the native aortic annulus).

Referring again to FIG. 9, though the illustrated embodiment shows three expansion mechanisms 404 spaced apart from each other about the circumference of the hybrid frame 402, it should be noted that any number of expansion mechanisms can be used. For example, in some embodiments, a prosthetic valve can comprise a single expansion mechanism, or two expansion mechanisms, or four expansion mechanisms, etc. The expansion mechanisms 404 can be placed at any position about the circumference of the frame 402. For example, in some embodiments, such as the illustrated embodiment, the expansion mechanisms 404 are equally spaced from one another about the circumference of the frame 402. In other embodiments, it can be advantageous to have two or more expansion mechanisms situated adjacent to one another.

Each expansion mechanism 404 can comprise an outer member 432 having an inner cavity or bore (not shown) and an inner member 434 extending at least partially into the bore. As shown in FIG. 10, a distal end portion 436 of the inner member 434 can be coupled to the first sub-frame 410 at a first location via a fastener 438 that is affixed to and extends radially from the distal end portion 436 of the inner member 434. The fastener 438 can be for example, a rivet or pin. As shown, in some embodiments, the fastener 438 can extend through corresponding apertures at a junction of two overlapping struts 414 of the first sub-frame 410 and can serve as a pivot pin around which the two struts 414 can pivot relative to each other and the inner member 434. In some embodiments, an end cap or nut can be disposed over an end portion of the fastener 438. The nut can have a diameter greater than the diameter of the apertures to retain the fastener 438 within the apertures. In alternative embodiments, the inner member 434 need not comprise a fastener 438 and can be coupled to the first sub-frame 410 via other means of attachment such as welding, adhesives, etc.

The outer member 432 can be coupled to the first sub-frame 410 at a second location, axially spaced from the first location. For example, in the illustrated embodiment, the inner member 434 is secured to the first sub-frame 410 near the distal or inflow end of the frame 406 and the outer member 432 is secured to the first sub-frame 410 closer to or at the proximal or outflow end 408 of the frame 402, such as via a fastener 438 (e.g., a rivet or pin). The fastener 438 is affixed to and extends radially from the outer member 432 through corresponding apertures at a junction of two overlapping struts 414 and can serve as a pivot pin around which the two struts 414 can pivot relative to each other and the outer member 432. As with fastener 438, a nut (not shown) mounted on fastener 438 to retain the fastener 438 within the corresponding apertures. In other embodiments, expansion mechanism 404 can be pivotably coupled to the frame at any two axially spaced, circumferentially aligned locations on the first sub-frame 410.

The inner member 434 can be axially movable relative to the outer member 432 in the proximal and distal directions. As such, because the inner member 434 and the outer member 432 are secured to the first sub-frame 410 at axially spaced locations, moving the inner member 434 and the outer member 432 axially with respect to one another in a telescoping manner can cause radial expansion or compression of the first sub-frame 410. For example, moving the inner member 434 proximally toward the outflow end 408 of the frame while holding the outer member 432 in a fixed position and/or moving the outer member 432 distally toward the inflow end 406 of the frame can cause the first sub-frame 410, and therefore the hybrid frame 402, to foreshorten axially and expand radially. Conversely, moving the inner member 434 distally and/or moving the outer member 432 proximally causes the first sub-frame 410, and therefore the hybrid frame 402, to elongate axially and compress radially.

In some embodiments, the expansion mechanism 404 can additionally be coupled to the second sub-frame 412. For example, the outer member 432 and/or the inner member 434 can comprise an additional fastener that is affixed to and extends radially from the outer member 432 or inner member 434. The additional fastener can be, for example, a rivet or a pin configured to extend into a corresponding aperture in the second sub-frame 412. The additional fastener can be slidable relative to the outer and/or inner members 432, 434, such that the fastener can slide proximally and/or distally as the hybrid frame 402 expands or contracts. In such embodiments, the expansion mechanism 404 can be coupled to the second sub-frame 412 at a third axially spaced location disposed between the first and second positions. As mentioned previously, in some embodiments, the expansion mechanism can be the means by which the first and second sub-frames 410, 412 are coupled together.

In use, the one or more expansion mechanisms 404 can be configured to move the prosthetic valve 400 between the radially compressed and radially expanded configurations by radially expanding and/or radially compressing the first sub-frame 410, and the prosthetic valve 400 can be retained at its functional size against forces applied by the patient's native annulus by the second sub-frame 412. This configuration does not require the expansion mechanisms 404 to include a locking component because the second sub-frame 412 serves to lock the hybrid frame 402 in the expanded configuration. The lack of a locking member can advantageously minimize the crimp profile of the prosthetic valve 400. Furthermore, the configuration can advantageously simplify manufacturing of the expansion mechanisms 404, for example, by allowing much simpler processing and machining procedures (such as Swiss-type and milling procedures) to be used. However, in some embodiments, a locking component may be included for additional security.

Further details of the expansion mechanisms and expansion and locking mechanisms can be found in International Application No. PCT/US2020/057691. In some embodiments, the expansion mechanisms 404 can be similar to those disclosed in International Application No. PCT/US2020/057691 but can exclude features that lock the frame in the expanded state. In other embodiments, the expansion mechanisms 404 can be the same as those disclosed in International Application No. PCT/US2020/057691.

The prosthetic valve 400 including hybrid frame 402 can be implanted in the following exemplary manner. Generally, the prosthetic valve 400 is placed in a radially compressed state and releasably coupled to a distal end portion of a delivery apparatus, such as delivery apparatus 300 (FIG. 8), and then advanced through the vasculature of a patient to a selected implantation site (e.g., the native aortic annulus). The prosthetic valve 400 can then be deployed at the implantation site and the first sub-frame 410 can be expanded using the expansion mechanisms 404 thereby expanding the prosthetic valve 400 to the expanded configuration. Once the prosthetic valve 400 has been implanted at the selected implantation site, the patient's native anatomy (e.g., the native aortic annulus) may exert radial forces against the prosthetic valve 400 that would tend to compress the frame 402. The second sub-frame 412 can exert sufficient force to prevent such forces from compressing the frame, thereby retaining the prosthetic valve 400 in the expanded configuration.

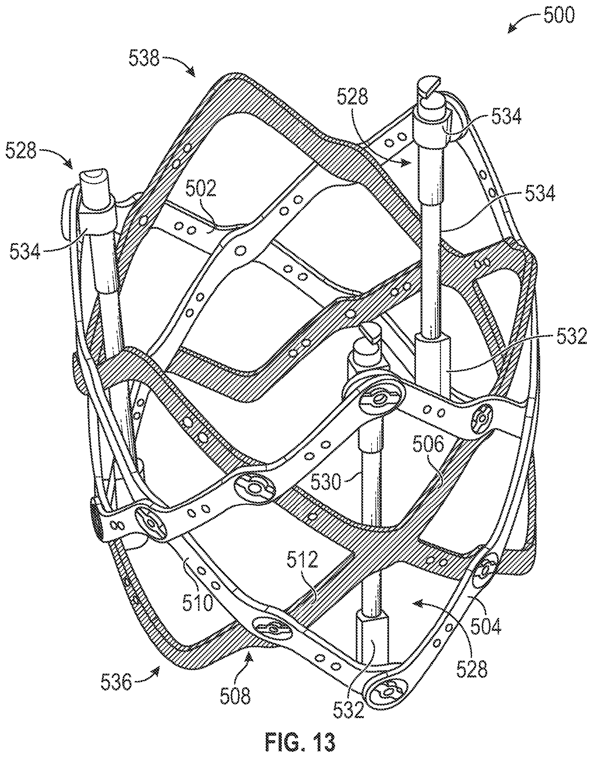

FIGS. 11A-13 illustrate another embodiment of a prosthetic heart valve 500 having a hybrid frame 502 (FIG. 13) comprising a first sub-frame 504 (FIG. 12) and a second sub-frame 506 (FIG. 11). Hybrid frame 502 can be similar to hybrid frame 402 (e.g., first sub-frame 504 can be a mechanically expanding sub-frame similar to sub-frame 410 and second sub-frame 506 can be a self-expanding sub-frame similar to sub-frame 412), except that the first and second sub-frames 504, 506 can be coupled together using hinges 508 integral to each sub-frame 504, 506, as described in more detail below.

As shown in FIGS. 11A-12B, the components forming the hinges 508 can be integrated into the construction of the respective struts 510, 512 of each sub-frame 504, 506. Referring to FIG. 11A, the struts 512 of the second sub-frame 506 can comprise a plurality of integral projections 514 spaced along the length of the strut 512 at the locations of the junctions 508. As best shown in FIG. 11B, each projection 514 can include a cylindrical base 516 and a locking member in the form of a plurality of ears 518 extending laterally from the base 516. In the illustrated embodiment, each projection includes two ears 518 that extend in opposite directions from the base 516, although in other embodiments any number of ears can be used and the ears can extend in any direction.

Referring to FIG. 12A, the struts 510 of the first sub-frame 504 can comprise a plurality of openings or apertures 520 spaced along the length of the strut at the location of junctions 508. As best shown in FIG. 12B, each opening 520 can include a central portion 522 and two oblong side portions 524 corresponding to the shape of the base 516 and ears 518, respectively. Each opening 520 can be formed within a recessed portion 526 formed on an outer surface of the strut 510.

When hybrid frame 502 is assembled, as shown in FIG. 13, the first sub-frame 504 can be positioned radially outwardly of the second sub-frame 506. The base 516 of each projection 514 extends through a corresponding opening 520, with the ears 518 residing in the recessed portion 526 surrounding the opening. The depth of the recessed portion 526 desirably is equal to or greater than the height of the ears 518 so that the projections do not extend radially beyond the outer surfaces of the outer struts 510. The ears 518 and the correspondingly shaped oblong side portions 524 allow the projection 514 to be inserted through the opening 520 when the ears 518 and the oblong side portions 524 are rotationally aligned with each other and then prevent separation of the two struts 510, 512 when the ears 518 and the side portions 524 are rotationally offset or misaligned with each other.

During assembly, the ears 518 of a strut 512 are aligned with the oblong side portions 524 of an opening 520 of a strut 510 corresponding to a predetermined angle between the struts which is greater than the maximum angle between the struts 510, 512. Thus, once the projections 514 of struts 512 are inserted through corresponding openings 520 of struts 510 to form the frame 502, the struts 510, 512 are then rotated relative to each other, which causes the ears 518 to become offset from the oblong side portions 524.

The hybrid frame 502 can comprise expansion mechanisms 528 mounted to the first sub-frame 504. The expansion mechanisms 528 can be similar to actuators 200 described above, except that expansion mechanisms 528 need not necessarily include a screw member. Each expansion mechanism 528 can comprise an inner member 530, a distal nut or sleeve 532 coupled to the sub-frame 504 at a first axial location, and a proximal nut or sleeve 534 coupled to the first sub-frame 504 at a second axial location spaced apart from the first. The inner member 530 can be slidable relative to the proximal sleeve 534 and coupled to the distal sleeve 532 such that proximal or distal motion of the inner member 534 causes proximal or distal motion of the inflow end portion 536 of the frame 502 relative to the outflow end portion 538 in order to expand and/or compress the frame. In some embodiments, the inner member 530 and the distal sleeve 532 can be formed integrally with one another. In other embodiment, the inner member 530 can comprise a threaded portion configured to couple a correspondingly threaded portion of the distal sleeve 532, which allows the inner member 530 to be removed from the distal sleeve 530 and the prosthetic valve after expansion of the prosthetic valve within a patient.

The inner member 530 can be axially movable relative to the proximal sleeve 534 in the proximal and distal directions. As such, because the inner member 530 is secured to the first sub-frame 504 via the distal sleeve 532 at a location axially spaced from the proximal sleeve 534, moving the inner member 530 and the proximal sleeve 534 axially with respect to one another in a telescoping manner can cause radial expansion or compression of the first sub-frame 504. For example, moving the inner member 530 proximally toward the outflow end 538 of the frame 502 while holding the proximal sleeve 534 in a fixed position and/or moving the proximal sleeve 534 distally toward the inflow end 536 of the frame can cause the first sub-frame 504, and therefore the hybrid frame 502, to foreshorten axially and expand radially. Conversely, moving the inner member 530 distally and/or moving the proximal sleeve 534 proximally causes the first sub-frame 504, and therefore the hybrid frame 502, to elongate axially and compress radially.

The expansion mechanisms 528 are configured to radially expand and contract the first sub-frame 504, but desirably limit the radial expansion and contraction of the frame 502 within a predetermined range of diameters and a predetermined range of angles between the struts 510, 512 at which the ears 518 are still rotationally offset from the oblong side portions 524. In this manner, the expansion mechanisms 528 can prevent radial expansion of the hybrid frame 502 to a diameter at which the ears 518 are rotationally aligned with the oblong side portions 524, thereby preventing separation of the struts 510, 512 at any of the junctions 508. Similarly, the expansion mechanisms 528 can prevent radial contraction of the frame to a diameter at which the ears 518 are rotationally aligned with the oblong side portions 524, thereby preventing separation of the struts 510, 512 at any of the junctions 508 when the frame is compressed to a delivery configuration.

The hinges 508 can be formed from the projections 514 and openings 520 having any of various shapes in addition to those shown in the illustrated embodiment. In general, the projections 514 can be formed with a locking member that has a non-circular shape (in a plane perpendicular to the central axis of the projection) and the openings 520 can have any non-circular shape that be rotationally aligned with the locking member to permit assembly of the struts and then rotationally offset from the locking member to prevent separation of the struts at the hinge. Further details regarding the hinges 508 and other types of hinges that can be implemented in the frame 502 are disclosed in U.S. Patent No. 10,869,759, which is incorporated herein by reference.

The prosthetic valve 500 including hybrid frame 502 can be implanted at a selected implantation site in the manner described above with respect to prosthetic valve 400.

Referring now to hybrid frames 400 and 500, in some embodiments, rather than exerting a radially outwardly directed force, the second sub-frame (e.g., sub-frame 412/506) can exert a radially inwardly directed force on the hybrid frame. In other words, rather than being a self-expanding sub-frame configured to bias the hybrid frame into the expanded configuration, the second sub-frame 412/504 can be a "self-contracting" sub-frame configured to bias the hybrid frame toward the compressed configuration.

In such embodiments, the expansion mechanisms 404/528 of the prosthetic valves 400/500 can be expansion and locking mechanisms including a locking member configured to retain the prosthetic valve in a radially expanded state. Further details regarding expansion and locking mechanisms can be found, for example, in International Application No. PCT/US2020/057691.

If repositioning or recapture and removal of the prosthetic valve is desired prior to locking, the self-contracting second sub-frame 412/506 can be allowed to contract (e.g., by releasing the force(s) applied by the one or more expansion and locking mechanisms) thereby causing radial compression of the hybrid frame 402/502 to a contracted free-state. When in the contracted free-state, the hybrid frame 402/502 can be between the fully expanded and fully compressed configurations. That is, when in the contracted free-state, the hybrid frame 402/502 is partially compressed and can have a diameter less than the fully expanded diameter, but greater than the fully compressed diameter, which typically requires the use of an external force (e.g., an actuator, an expansion mechanism etc.) to achieve. For example, when in the fully compressed configuration the frame 402/502 can have a diameter of between about 6 mm and about 7 mm, and when in the contracted free-state the frame 402/502 can have a diameter of between about 10 mm and about 12 mm. In alternative embodiments, the contraction force of the self-contracting sub-frame can be sufficient to compress the hybrid frame to the fully compressed state.

The contraction force exerted by the second sub-frame 412/506 can be sufficient to overcome the forces associated with the prosthetic heart valve's soft components (e.g., the valvular structure 18). The contraction force can also be small enough that it does not prevent the expansion mechanisms 404/528 from expanding the frame 402/502.

A prosthetic valve 400/500 comprising a self-contracting second sub-frame 412/506 can be implanted in the following exemplary manner. Generally, the prosthetic valve is placed in a radially compressed state and releasably coupled to a distal end portion of a delivery apparatus, such as delivery apparatus 300 (FIG. 8), and then advanced through the vasculature of a patient to a selected implantation site (e.g., the native aortic annulus). The prosthetic valve 400/500 can then be deployed at the implantation site and expanded using the expansion and locking mechanisms. If repositioning or removal of the prosthetic valve is desired, the second sub-frame 412/506 can be allowed to self-contract to compress the hybrid frame 402/502 to the contracted free-state. For example, the second sub-frame 412/506 can self-contract once the force applied by the expansion and locking mechanisms is removed. When in the contracted free-state, the frame can have a diameter smaller than that of the patient's native annulus, allowing the prosthetic valve 400/500 to be more easily repositioned within the annulus.

Once repositioned, the expansion and locking mechanisms can be used to expand the first sub-frame 410/504, thereby expanding the hybrid frame 402/502 and lock the frame 402/502 to the expanded configuration.

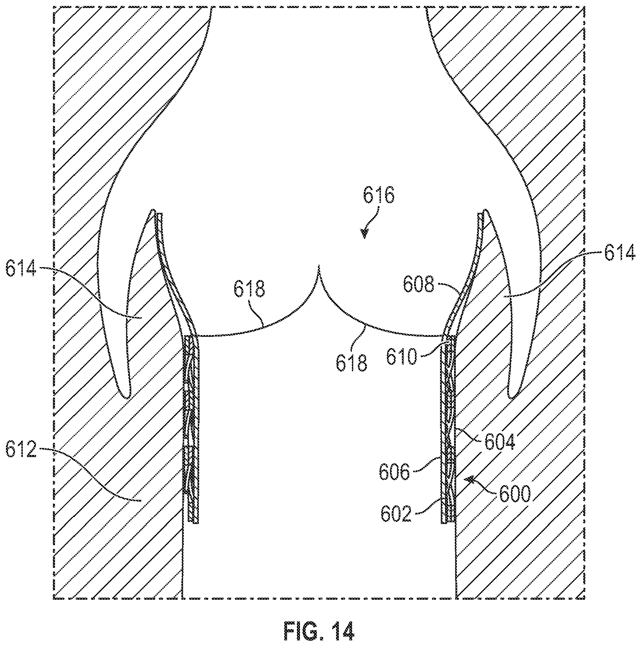

FIG. 14 illustrates an exemplary embodiment of a prosthetic heart valve 600 comprising a hybrid frame 602 that includes first and second sub-frames 604, 606. In some embodiments, the first and second sub-frames 604 and 606 are similar to first and second sub-frames 410 and 412 described above, except that second sub-frame 606 is axially longer than first sub-frame 604, such that an extending portion 608 of second sub-frame 606 extends beyond the outflow end portion 610 of first sub-frame 604. FIG. 14 shows the prosthetic heart valve 600 implanted within the native aortic valve for purposes of illustration.

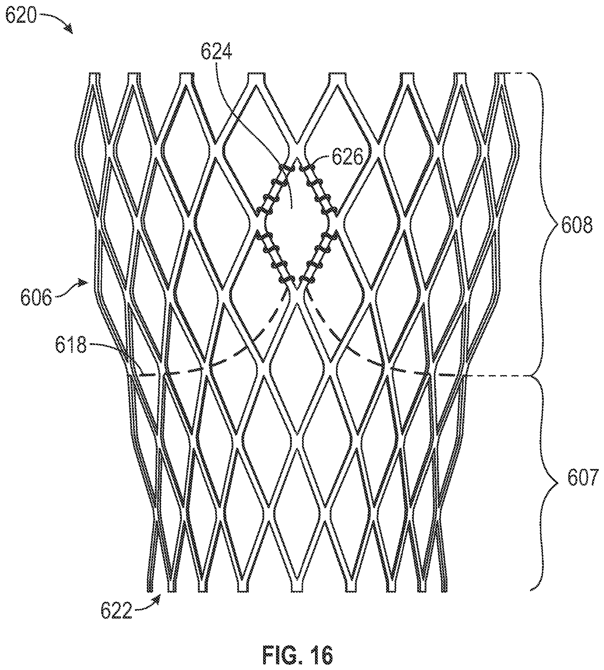

In particular embodiments, the first sub-frame 604 comprises the frame 12 of FIG. 1 and the second sub-frame 606 comprises the frame configuration shown in FIG. 16. The first and second sub-frames 604, 606 can be connected to each other using mechanical fasteners, sutures, welding, adhesives, etc., as previously described.

As shown in FIG. 16, in some embodiments, the frame 606 can have an annulus portion 607 and an extending portion 608 having a flared shape. That is, a diameter of the outflow end portion 620 can be larger than a diameter of the inflow end portion 622. The frame 606 can be laser cut from a metal tube, as known in the art.

As shown in FIG. 14, this configuration allows the prosthetic heart valve 600 to be deployed such that the first sub-frame is anchored within the native aortic annulus 612, while the extending portion 608 of the second sub-frame 606 can be used to hold open the native leaflets 614. In some embodiments, the extending portion 606 can be shape-set to form a particular shape, for example, in some embodiments, the extending portion can be shape-set to form a clover-shaped cross-sectional profile in a plane perpendicular to a central longitudinal axis of the prosthetic valve.

The hybrid frame 602 can comprise one or more expansion mechanisms (not shown), similar to expansion mechanisms 404 or 528 described above. The expansion mechanisms can be coupled to the first sub-frame 604 and can be devoid of locking mechanisms, which can advantageously reduce the crimping profile of the prosthetic heart valve 600. As described previously with respect to hybrid frame 402, the expansion mechanisms can be used to move the first sub-frame 604 between the radially compressed configuration and the radially expanded configuration and the second sub-frame 606 can generate sufficient force to retain the hybrid frame 602 in the expanded configuration against the forces exerted by the native anatomy.

In the illustrated embodiment, the first sub-frame 604 is the radially outer sub-frame, and the second sub-frame 606 is the radially inner sub-frame. In such embodiments, the valvular structure 616 can be coupled to the second sub-frame 604. As shown in FIG. 16, the leaflets 618 of the valvular structure 616 can be secured to one another at adjacent sides to form commissures 624, each of which can be secured to the second sub-frame 606 using, for example, a plurality of sutures 626.

In particular embodiments, the valvular structure 616 can be coupled to the extending portion 608, such as shown in FIG. 16. Coupling the valvular structure 616 to the Nitinol sub-frame 606 can improve hemodynamic flow through the prosthetic valve 600. For example, the flexibility of the Nitinol sub-frame 606 can help improve the hemodynamics of the prosthetic valve by acting as a force dampener and thereby reducing the force load on the leaflets 618. Additionally, since the second sub-frame 606 is thinner than the first sub-frame 604, attaching the valvular structure to the extending portion 608 of the second sub-frame 606 can advantageously minimize the loss of conduit diameter (e.g., the inner diameter of the prosthetic valve), and also provide a thinner profile for the prosthetic heart valve when in the compressed configuration.

This configuration can also provide advantages during the assembly of the prosthetic valve 600. The valvular structure 616 can be coupled to the second sub-frame 606 prior to coupling the second sub-frame 606 to the first sub-frame 604, which simplifies the assembly process.

Alternatively, in other embodiments, the first sub-frame 604 can be the inner sub-frame, and the valvular structure 616 can be coupled to the first sub-frame 604.

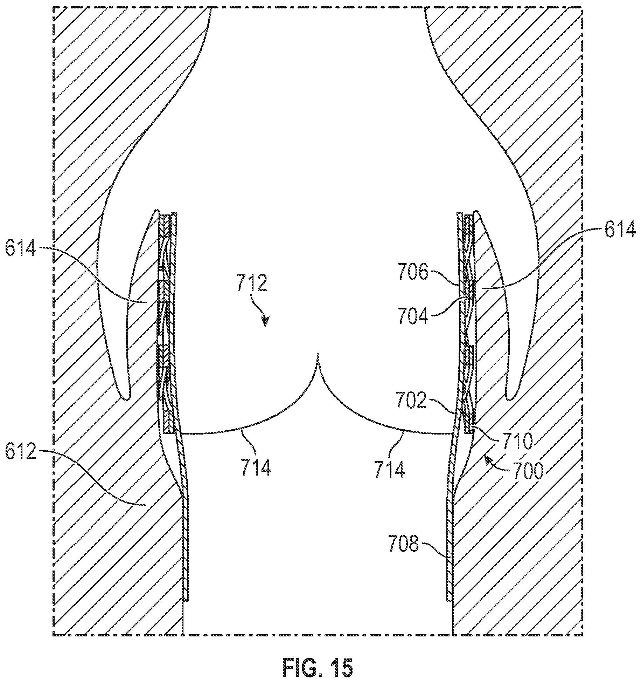

FIG. 15 illustrates an exemplary embodiment of a prosthetic heart valve 700 comprising a hybrid frame 702 that includes first mechanically expandable sub-frame 704 and second self-expanding sub-frame 706. In some embodiments, the first and second sub-frames 704 and 706 are similar to first and second sub-frames 604 and 606 described above, except that the extending portion 708 of second sub-frame 706 extends distally beyond the inflow end portion 710 of first sub-frame 704.

As shown in FIG. 15, this configuration allows the prosthetic heart valve 700 to be deployed such that the first sub-frame is mounted above the native aortic annulus 612 to wedge the native leaflets 614 open and allows the extending portion 708 of the second sub-frame 706 to be deployed within the native annulus 612. The lower forces exerted by the second sub-frame 706 can advantageously mitigate the risk of annular rupture. Furthermore, the second sub-frame can better conform to the anatomical shape of the native annulus when expanded therein, thereby optimizing valve conduit geometry. The relatively greater forces exerted by the first sub-frame 704 can advantageously be used to wedge open the native leaflets 614, particularly in situations where the native leaflets 614 have become calcified.

The hybrid frame 702 can comprise one or more expansion mechanisms (not shown), similar to expansion mechanisms 404 or 528 described above. The expansion mechanisms can be coupled to the first sub-frame 704 and can be devoid of locking mechanisms, which can advantageously reduce the crimping profile of the prosthetic heart valve 702. As described previously with respect to hybrid frame 402, the expansion mechanisms can be used to move the first sub-frame 704 between the radially compressed configuration and the radially expanded configuration. The second sub-frame 706 can generate sufficient force to retain the frame 702 in the expanded configuration against the forces exerted by the native anatomy.

In the illustrated embodiment, the first sub-frame 704 is the outer sub-frame, and the second sub-frame 706 is the inner sub-frame. In such embodiments, the valvular structure 712 can be coupled to the second sub-frame 706. In particular embodiments, the valvular structure 712 can be coupled to the extending portion 708. As mentioned previously, coupling the valvular structure 712 to the Nitinol sub-frame 706 can improve hemodynamic flow through the prosthetic valve 700. For example, the flexibility of the Nitinol sub-frame 706 can help improve the hemodynamics of the prosthetic valve 700 by acting as a force dampener and thereby reducing the force load on the leaflets 714. Additionally, since the second sub-frame 706 is thinner than the first sub-frame 704, attaching the valvular structure 712 to the extending portion 708 of the second sub-frame 706 can advantageously minimize the loss of conduit diameter (e.g., the inner diameter of the prosthetic valve), and also provide a thinner profile for the prosthetic heart valve 700 when in the compressed configuration.

This configuration can also provide advantages during the assembly of the prosthetic valve 700. The valvular structure 712 can be coupled to the second sub-frame 706 prior to coupling the second sub-frame 706 to the first sub-frame 704 which simplifies the assembly process.

Alternatively, in other embodiments, the first sub-frame 704 can be the inner sub-frame, and the valvular structure can be coupled to the first sub-frame 704. In other embodiments, the first sub-frame 704 can be the inner sub-frame and the second sub-frame 706 can be the outer sub-frame and the valvular structure 712 can be coupled to the extending portion 708 of the second sub-frame 706 at a location distal to the inflow end 710 of the first sub-frame 704.

## Claims

1. An implantable prosthetic device (400), comprising:
a hybrid frame (402) movable between a radially compressed configuration and a radially expanded configuration, the hybrid frame (402) comprising:
a mechanically-expandable first sub-frame (410) comprising a plurality of struts (414) pivotably coupled to one another, and
a self-expanding second sub-frame (412) coupled to the first sub-frame (410);
wherein, when the hybrid frame (402) is in the expanded configuration, the second sub-frame (412) is configured to resist radial compression of the frame (402); and
wherein the second sub-frame (412) is configured to exert a radially outwardly directed force on the first sub-frame (410) to resist radial compression of the first sub-frame (410).

2. The implantable prosthetic device (400) of claim 1, wherein the second sub-frame (412) is disposed radially inwardly of the first sub-frame (410).

3. The implantable prosthetic device (400) of any of claims 1-2, further comprising one or more expansion mechanisms (404) coupled to the first sub-frame (410), the expansion mechanisms (404) configured to move the first sub-frame (410) between the radially compressed configuration and the radially expanded configuration.

4. The implantable prosthetic device (400) of claim 3, wherein each expansion mechanism (404) comprises a first member (432) coupled to the first sub-frame (410) at a first location and a second member (434) coupled to the first sub-frame (410) at a second location spaced apart from the first location, the second member (434) extending at least partially into the first member (432).

5. The implantable prosthetic device (400) of any of claims 3-4, wherein the first and second sub-frames (410, 412) are coupled to one another via the one or more expansion mechanisms (404).

6. The implantable prosthetic device (400) of any of claims 1-5, wherein the second sub-frame (412) is formed as a unitary piece of material.

7. The implantable prosthetic device (400) of any of claims 1-6, wherein a plurality of projections (514) are formed on the second sub-frame (412) and coupling the second sub-frame (412) to the first sub-frame (410) comprises inserting the projections (514) through corresponding apertures (520) in the first sub-frame (410).

8. The implantable prosthetic device (400) of any of claims 1-7, further comprising a valvular structure (18) including a plurality of leaflets (20), the valvular structure (18) coupled to the second sub-frame (412).

9. The implantable prosthetic device (400) of any of claims 3-7, further comprising a valvular structure (18) including a plurality of leaflets (20), the valvular structure (18) coupled to the one or more expansion mechanisms (404).

## Patentansprüche

1. Implantierbare Prothesenvorrichtung (400) umfassend:
einen Hybridrahmen (402), der zwischen einer radial komprimierten Konfiguration und einer radial expandierten Konfiguration beweglich ist, wobei der Hybridrahmen (402) umfasst:
einen mechanisch expandierbaren ersten Teilrahmen (410), der eine Vielzahl von Streben (414) umfasst, die schwenkbar aneinander gekoppelt sind, und
einen selbstexpandierenden zweiten Teilrahmen (412), der an den ersten Teilrahmen (410) gekoppelt ist;
wobei, wenn der Hybridrahmen (402) in der expandierten Konfiguration vorliegt, der zweite Teilrahmen (412) dafür gestaltet ist, radialer Kompression des Rahmens (402) zu widerstehen; und
wobei der zweite Teilrahmen (412) dafür gestaltet ist, eine radial nach außen gerichtete Kraft auf den ersten Teilrahmen (410) auszuüben, um radialer Kompression des ersten Teilrahmens (410) zu widerstehen.

2. Implantierbare Prothesenvorrichtung (400) nach Anspruch 1, wobei der zweite Teilrahmen (412) radial nach innen bezogen auf den ersten Teilrahmen (410) angeordnet ist.

3. Implantierbare Prothesenvorrichtung (400) nach einem der Ansprüche 1-2, ferner umfassend einen oder mehrere Expansionsmechanismen (404), die an den ersten Teilrahmen (410) gekoppelt sind, wobei die Expansionsmechanismen (404) dafür gestaltet sind, den ersten Teilrahmen (410) zwischen der radial komprimierten Konfiguration und der radial expandierten Konfiguration zu bewegen.

4. Implantierbare Prothesenvorrichtung (400) nach Anspruch 3, wobei jeder Expansionsmechanismus (404) ein erstes Element (432), das an einer ersten Stelle an den ersten Teilrahmen (410) gekoppelt ist, und ein zweites Element (434), das an einer zweiten Stelle mit Abstand von der ersten Stelle an den ersten Teilrahmen (410) gekoppelt ist, umfasst, wobei sich das zweite Element (434) wenigstens teilweise in das erste Element (432) erstreckt.

5. Implantierbare Prothesenvorrichtung (400) nach einem der Ansprüche 3-4, wobei der erste und der zweite Teilrahmen (410, 412) über den einen oder die mehreren Expansionsmechanismen (404) aneinander gekoppelt sind.

6. Implantierbare Prothesenvorrichtung (400) nach einem der Ansprüche 1-5, wobei der zweite Teilrahmen (412) als als einstückiges Materialstück gestaltet ist.

7. Implantierbare Prothesenvorrichtung (400) nach einem der Ansprüche 1-6, wobei eine Vielzahl von Vorsprüngen (514) an dem zweiten Teilrahmen (412) gebildet sind und Koppeln des zweiten Teilrahmens (412) an den ersten Teilrahmen (410) Einführen der Vorsprünge (514) durch entsprechende Öffnungen (520) in dem ersten Teilrahmen (410) umfasst.

8. Implantierbare Prothesenvorrichtung (400) nach einem der Ansprüche 1-7, ferner umfassend eine Klappenstruktur (18), die mehrere Segel (20) aufweist, wobei die Klappenstruktur (18) an den zweiten Teilrahmen (412) gekoppelt ist.

9. Implantierbare Prothesenvorrichtung (400) nach einem der Ansprüche 3-7, ferner umfassend eine Klappenstruktur (18), die mehrere Segel (20) aufweist, wobei die Klappenstruktur (18) an den einen oder die mehreren Expansionsmechanismen (404) gekoppelt ist.

## Revendications

1. Dispositif prothétique implantable (400) comprenant :
un bâti hybride (402) mobile entre une configuration comprimée radialement et une configuration déployée radialement, le bâti hybride (402) comprenant :
un premier sous-bâti (410) mécaniquement expansible comprenant une pluralité d'entretoises (414) couplées de manière pivotante les unes aux autres, et
un second sous-bâti (412) auto-expansible couplé au premier sous-bâti (410),
lorsque le bâti hybride (402) est en configuration déployée, le second sous-bâti (412) étant configuré pour résister à la compression radiale du bâti (402) ; et
le second sous-bâti (412) étant configuré pour exercer une force dirigée radialement vers l'extérieur sur le premier sous-bâti (410) afin de résister à la compression radiale du premier sous-bâti (410).

2. Dispositif prothétique implantable (400) selon la revendication 1, le second sous-bâti (412) étant disposé radialement vers l'intérieur du premier sous-bâti (410).

3. Dispositif prothétique implantable (400) selon l'une quelconque des revendications 1 et 2, comprenant en outre un ou plusieurs mécanismes d'expansion (404) couplés au premier sous-bâti (410), les mécanismes d'expansion (404) étant configurés pour déplacer le premier sous-bâti (410) entre la configuration comprimée radialement et la configuration déployée radialement.

4. Dispositif prothétique implantable (400) selon la revendication 3, chaque mécanisme d'expansion (404) comprenant un premier élément (432) couplé au premier sous-bâti (410) à un premier emplacement et un second élément (434) couplé au premier sous-bâti (410) à un second emplacement espacé du premier emplacement, le second élément (434) s'étendant au moins partiellement dans le premier élément (432).

5. Dispositif prothétique implantable (400) selon l'une quelconque des revendications 3 et 4, les premier et second sous-bâtis (410, 412) étant couplés l'un à l'autre par l'intermédiaire du ou des mécanismes d'expansion (404).

6. Dispositif prothétique implantable (400) selon l'une quelconque des revendications 1 à 5, le second sous-bâti (412) étant formé comme une pièce unitaire de matériau.

7. Dispositif prothétique implantable (400) selon l'une quelconque des revendications 1 à 6, une pluralité de saillies (514) étant formées sur le second sous-bâti (412) et couplant le second sous-bâti (412) au premier sous-bâti (410) comprenant l'insertion des saillies (514) à travers des ouvertures correspondantes (520) dans le premier sous-bâti (410).

8. Dispositif prothétique implantable (400) selon l'une quelconque des revendications 1 à 7, comprenant en outre une structure valvulaire (18) comprenant une pluralité de feuillets (20), la structure valvulaire (18) étant couplée au second sous-bâti (412).

9. Dispositif prothétique implantable (400) selon l'une quelconque des revendications 3 à 7, comprenant en outre une structure valvulaire (18) comprenant une pluralité de feuillets (20), la structure valvulaire (18) étant couplée au ou aux mécanismes d'expansion (404).
